# EUROPEAN PATENT APPLICATION

(11) **EP 4 339 272 A1**
(43) Date of publication of application: **20.03.2024**
(21) Application number: 22195280.7
(22) Date of filing: 13.09.2022
(51) Int. Cl.: C12M 1/00, C12M 1/42

(54) **SYSTEMS AND METHODS FOR DEVELOPING AND OPTIMIZING CELL CULTURE PROCESSES**

(71) Applicant: The Automation Partnership (Cambridge) Ltd., Hertfordshire SG8 5WY (GB)
(72) Inventor: Betts, John, London N4 4BX (GB)
(74) Representative: Michalski Hüttermann & Partner Patentanwälte mbB

(57) **Abstract**

The present application relates to the field of development and optimization of cell culture processes for production of biotechnological and biopharmaceutical products. The present invention particularly relates to systems comprising at least a cell culturing device and a cell sorting device, and optionally a direct connection from the cell culturing device to the cell sorting device, and a direct connection from the cell sorting device to the cell culturing device. The invention further relates to methods for developing and/or optimizing a cell culture process, comprising at least the steps of growing a cell culture of a polyclonal population of cells in a cell culturing device and selecting cells in the cell sorting device, and optionally transferring cells from the cell culturing device into a cell sorting device by direct connection(s) from the cell culturing device to the cell sorting device, and recirculating selected cells from the cell sorting device back to the cell culturing device by direct connection(s) from the cell sorting device to the cell culturing device

## Description

### Field of the invention

The present application relates to the field of development and optimization of cell culture processes for production of biotechnological and biopharmaceutical products. The present invention particularly relates to systems comprising at least a cell culturing device and a cell sorting device, and optionally a direct connection from the cell culturing device to the cell sorting device, and optionally a direct connection from the cell sorting device to the cell culturing device. The invention further relates to methods for developing and/or optimizing a cell culture process, comprising at least the steps of growing a cell culture of a polyclonal population of cells in a cell culturing device and selecting cells in the cell sorting device, and optionally transferring cells from the cell culturing device into a cell sorting device by direct connection(s) from the cell culturing device to the cell sorting device, and optionally recirculating selected cells from the cell sorting device back to the cell culturing device by direct connection(s) from the cell sorting device to the cell culturing device.

### Background

Over past decades, the development of industrial manufacturing of protein-based and cell-based biopharmaceuticals in cell culture systems has made tremendous progress and plays a huge role in health care management worldwide. In the last years, the biopharmaceutical industry has significantly turned its biologics production towards mammalian cell expression systems, in particular because of the presence of glycosylation machineries within these cells, and the fact that monoclonal antibodies represent today the vast majority of new therapeutic candidates, which has largely influenced this direction (Lalonde and Durocher 2017).

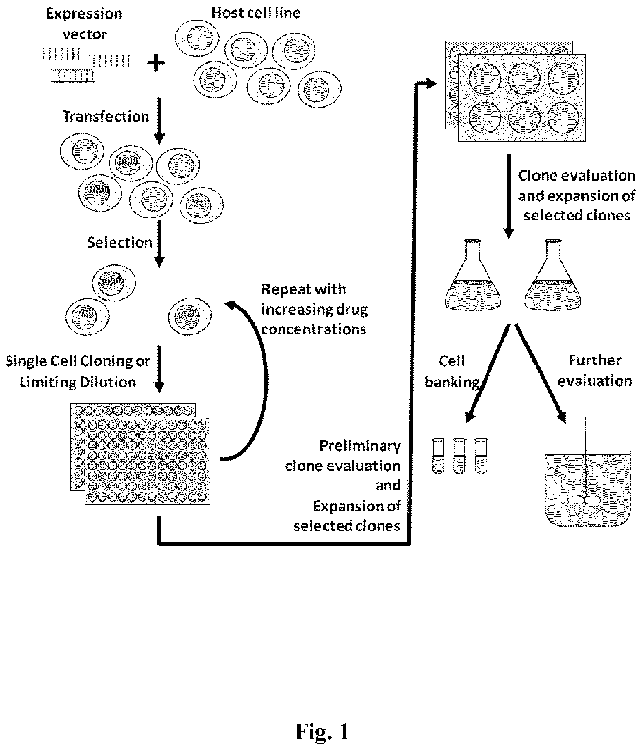

The development of a manufacturing process for a recombinant protein in mammalian cells usually follows a scheme comprising several steps (Fig. 1). Initially, the recombinant gene with the necessary transcriptional regulatory elements is transferred to the cells. Usually, in addition, a second gene is transferred that confers to recipient cells a selective advantage. After transfection of the host cell line with the expression vector(s) containing the gene of interest and the selection marker, the cells undergo drug selection and cloning to derive cells that are producing the polypeptide or protein of interest.

In the presence of the selection agent, only those cells that express the selector gene survive. Popular genes for selection are for example dihydrofolate reductase (DHFR), an enzyme involved in nucleotide metabolism, and glutamine synthetase (GS). In both cases, selection occurs in the absence of the appropriate metabolite (hypoxantine and thymidine, in the case of DHFR, glutamine in the case of GS), preventing growth of untransformed cells. For efficient expression of the recombinant protein, the gene encoding the biopharmaceutical and selector genes can be on the same plasmid or on different plasmids.

When gene amplification systems are used, concentrations of the selection drug can be increased step-wise to derive cell clones that are more productive. For example, methotrexate (MTX) inhibits dihydrofolate reductase, so selection is performed by culturing the cells in a selection medium lacking hypoxanthine and thymidine; low concentrations of MTX are then used to amplify the transfected genes for increased protein expression. Methionine sulfoximine (MSX) is a small molecule compound that serves as a selection reagent for clone generation with a glutamine synthetase.

Further selectable markers often used in mammalian expression vectors are shown in the table below (Li *et al.* 2010).

| **Selectable Marker** | **Selection Reagent** |
|---|---|
| **Metabolic Selectable Marker** | |
| Dihydrofolate reductase (DHFR) | Methionine sulphoximine (MSX) |
| Glutamine synthase (GS) | Methotrexate (MTX) |

| **Antibiotic Selectable Marker** | |
|---|---|
| Puromycin acetyltransferase | Puromycin |
| Blasticidin deaminase | Blastcidin |
| Histidinol dehydrogenase | Histidinol |
| Hygromycin phosphotransferase | Hygromycin |
| Zeocin resistance gene | Zeocin |
| Bleomycin resistance gene | Bleomycin |
| Aminoglycoside phosphotransferase | Neomycin (G418) |

Following selection, survivors are transferred as single cells to a second cultivation vessel, and the cultures are expanded to produce clonal populations (single-cell cloning). Eventually, individual clones are evaluated for recombinant protein expression, with the highest producers being retained for further cultivation and analysis. From these candidates, cell clones with high recombinant protein titer are chosen for progressive expansions before cell banking and further clone evaluations, such as production stability of the cell clones and quality of recombinant protein (Wurm 2004; Lai *et al.* 2013; Li *et al.* 2010).

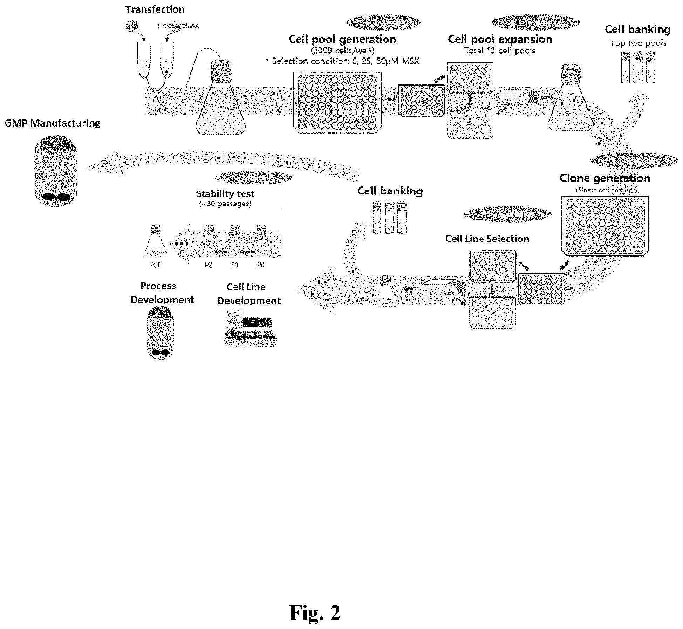

One cell line with the appropriate growth and productivity characteristics is chosen for production of the recombinant protein. A cultivation process is then established that is determined by the production needs ("bioprocessing"). Usually, mammalian recombinant therapeutics are naturally secreted proteins or have been developed from gene constructs that mediate protein secretion (Fig. 2).

Currently in the field of bioprocessing a large amount of effort, cost and time is expended screening genetically modified cells and cell lines to try and find those clones which are most suited to an industrial process. With current molecular biology techniques, DNA integration is largely a random process, so that as a result hundreds to thousands of clonal populations have to be screened to find the best-suited clone(s), wherein the criteria for selecting best clones may vary, but generally focus on cell performance predominantly concerning cell growth and stability, viability, metabolism, productivity, and product quality. Clone generation and selection is not optimised, and clones that appear promising during cell line selection may not perform well at typical process development or manufacturing scales of operation. One of the key reasons for this is because the engineering environments experienced by the cells in the selection stages (often uncontrolled process conditions, static or shaken, batch culture systems) are vastly different from those in development and manufacturing stages (typically controlled process conditions, stirred, fed-batch or continuous culture systems).

An inability to pick a high performing, industrially relevant clone introduces a level of risk and uncertainty into the process of developing a new biopharmaceutical which can slow down development timelines and impact on development costs. More importantly however, a clone that performs poorly at industrial scale may result in unsatisfactory safety and efficacy profiles, and in the worst cases bring a halt to the clinical development or commercial launch of a new drug product. With an increased pressure on biopharmaceutical companies to deliver cost effective therapeutics, the knock-on effect for cell line development teams in industry are for quicker development times, higher productivity cell lines, better product quality molecules, and more robust manufacturing processes.

A particular response to the challenges discussed above is to screen even more clones at the earliest stages of cell line development. Companies have attempted to address this by facilitating the screening of cell lines at higher throughputs, e.g., screening clones manually in microwell plate formats, or employing bespoke solutions to automate those processes, and suppliers have developed high-throughput screening platform products, e.g., BioProcessors SimCell or Berkley Lights Beacon system. Critically however, none of these systems adequately recreate the engineering environment of a typical manufacturing-scale culture system and therefore have limited utility in screening through the population of modified cells to find the best clones.

However, even if a perfect screening system was to exist, this would still not fully address the issue, as the root of the problem also lies prior to the cell line screening stage, at the clone generation, *i.e.* DNA integration, part of the process. Similar to the issues raised with the typical screening systems employed, DNA integration is routinely performed on cells grown under uncontrolled process conditions in static T flask cultures, *i.e.,* a biological and physical environment that is not consistent with the industrial-scale application.

Traditionally, production cell lines are generated by random integration of the product transgene into the host cell followed by gene amplification and screening for high producing cell clones. Although this method of cell line generation has been in practice for over three decades and such cell lines have been used to produce the majority of the products on the market, the success of the method relies on screening a large number of clones for their productivity and stability over time (O'Brien *et al.* 2018).

Albeit DNA integration is largely considered to be a random event, there is a propensity for foreign DNA to integrate near transcriptionally active DNA (Scherdin *et al.* 1990). The profile of genes that are actively expressed by cells in a bioreactor culture, for example, will be different than that for cells grown in a distinct culture format, *e.g.,* a microwell plate or shake flask. Therefore, by conducting the DNA integration step in physical and biological environment "A", the probability for a gene to be integrated into an area of the host genome that is *also* active in physical and biological environment "B" decreases notably; where A might be a low cell density, 100 mL static T-flask batch culture with uncontrolled process conditions, and B might be a high cell density, 100 L stirred tank reactor fed-batch culture with controlled process conditions, for example.

Hence, the objective of the present invention is to provide a means by which the DNA integration and subsequent pre-selection of modified cells can be done in a biological and physical environment that is consistent with that seen in a typical manufacturing scale environment, thus providing access to a means of creating pools of high-performing, industrially relevant modified clones which would otherwise not currently be possible, as well as to provide a respective method for developing and optimizing a cell culture process. Such means can be expected to significantly improve the selection for high yielding and stable production cell lines.

Further objectives of the present invention are to provide respective means and methods for developing and optimizing cell culture processes saving costs, time and resources as compared to currently employed processes.

Flow cytometry has been developed to become a versatile and powerful tool for identifying, analyzing and separating different types or populations of cells, for example, endothelial cells from monocytes (Umeyama et al. 2020), in basic research, cytology, immunology, oncology, and other disciplines. Multi-color flow cytometry has been shown to be suitable for determining the cell-type specific response of cell populations within an *in vitro* multicellular co-culture model, providing reliable data correlative to *in vivo* conditions (Clift *et al.* 2017).

Flow cytometry has also been used to optimize *in vitro* animal cell culture processes (Al-Rubeai and Emery, 1993). In flow cytometry, most often employed as fluorescence-activated cell cytometry, cells or other particles in suspension flow in single file at uniform speeds through a laser light beam with which they interact individually. This yields, for each cell, a light scatter pattern which provides information about cell size, shape, density and surface morphology. Furthermore, fluorophore labeling of cells and measurement can give quantitative data on specific target molecules or subcellular constituents and their distribution in the cell population (Al-Rubeai and Emery, 1993). Hence, flow cytometry has been widely used for cell culture monitoring and improvement, by measuring the response of the cell population to external stimuli such as nutrients, pH, temperature or hydrodynamic forces under changing cell culture conditions, and thus cell performance in the culture over time, and by adapting the culture conditions according to the data obtained. That way, cell growth, stability and productivity of the cell culture process can be optimized. Furthermore, any measurable property featured by the cells can be used as a basis for selection of individual cells, by physical sorting.

As disclosed in DE60308093T2, a light-reactive composition was developed whose cell-binding capacity varies with light irradiation and which can be used for providing a cell-adhering surface for growing anchorage-dependent cells in a cell culture process. The adherence of the cells to a cell culture device comprising said light-reactive composition can be used to regulate the attachment and detachment on basis of the level of light irradiation, and thus can be used also for cell separation.

US9149806B2 discloses a method for high-throughput cell sorting based on on-the-fly flow based field potential sensing, the method comprising stimulating a cell, sensing field potential signals of the cell as the cell flows through an array of spatially located electrodes after the cell being stimulated, and then identifying a cellular phenotype of the cell based on the field potential signals sensed from the array of spatially located electrodes.

According to the disclosure in EP2665807B1, a bioreactor is provided which is composed of three different compartments, separated by selectively permeable membranes, serving to isolate a particular cell population from many other kinds of cells derived from original samples introduced into the bioreactor, and then serving to efficiently expanding the isolated cell population and differentiating the isolated cells in the bioreactor culture.

Notwithstanding some rare examples of combining certain aspects of cell separation and propagation in the documents referred to above, the prior art does not disclose or suggest a system and method for developing and/or optimizing a cell culture process, in particular a cell culture process suited for large-scale manufacturing, and the use of the system in said method, according to the present invention. In particular, the concept of recirculating cells which have been enriched with regard to a particular cell type or cell clone by passing through a cell sorting device, back into the cell culturing device, according to the present invention has not been disclosed or suggested in the prior art.

### Summary of the Invention

The present invention provides means and methods for development and/or optimization of cell culture processes for production of biotechnological and biopharmaceutical products, solving the technical problems described above.

The present invention in particular provides systems comprising at least a cell culturing device and a cell sorting device. The systems may further comprise a direct connection from the cell culturing device to the cell sorting device, and a direct connection from the cell sorting device to the cell culturing device. The present invention further provides methods for developing and/or optimizing a cell culture process, comprising at least the steps of growing a cell culture of a polyclonal population of cells in a cell culturing device and selecting cells in the cell sorting device. The methods may further comprise transferring cells from the cell culturing device into a cell sorting device by direct connection(s) from the cell culturing device to the cell sorting device, and recirculating selected cells from the cell sorting device back to the cell culturing device by direct connection(s) from the cell sorting device to the cell culturing device.

In one embodiment, the present invention relates to a system in the form of an industrially relevant cell culture scale-down model, *e.g.,* an Ambr or Biostat system, and integrating into that system a device for actively selecting target cell populations, e.g., fluorescence- or magnetic-activated cell sorting (FACS, MACS). The manufacturing scale process could be translated to operation at the small scale using traditional means of scaling based on matched parameters, such as K_{L}a or mixing time, or by using tools such as that offered by the Process Insights application. In either case, the bioreactor could be operated in a manner that is reflective of the target industrial scale application, *i.e.,* a comparable physical and biological environment.

Having established the host cell line in culture conditions that are consistent with manufacturing-scale operation, the gene of interest can then be introduced to enable DNA integration. A recirculating loop, from the bioreactor via a cell sorting device can then be employed in a continuous or semi-continuous manner to enrich the heterogeneous pool of transfected cells in the culture vessel with desirable clones. This could be carried out until the pool of clones within the system has reached some target performance, *i.e.,* a measure of productivity or quality. At this point, the cell sorting device could now be used for an output function, selectively sorting cells into single cell populations for subsequent clone screening and/or cell banking.

In an embodiment of the present invention, the cell culturing device is a multi-parallel arrangement which could then be used to generate and select clones across a range of therapeutic molecules/host strains or process conditions, such as for example feeding regime, stir speed, media exchange rate in a perfusion process, or pH set point.

The solution to the technical problem according to the present invention can be employed for the generation and selection of stable cell lines expressing protein-based biopharmaceutical targets, e.g., monoclonal antibodies, but is also applicable for other applications, such as for example stable cells lines for viral vector production or allogeneic engineered immune cells.

Said and further objects are met with means and methods according to the independent claims of the present invention. The dependent claims are related to specific embodiments.

The invention and general advantages of its features will be discussed in detail below.

### Description of the Figures

**Fig. 1****.** Schematic illustration of a typical process to develop a mammalian cell line for recombinant protein manufacturing (from Lai *et al.* 2013).
**Fig. 2****.** Schematic illustration of a typical workflow for the establishment of recombinant CHO cells producing monoclonal antibodies (modified from Noh *et al.* 2018).

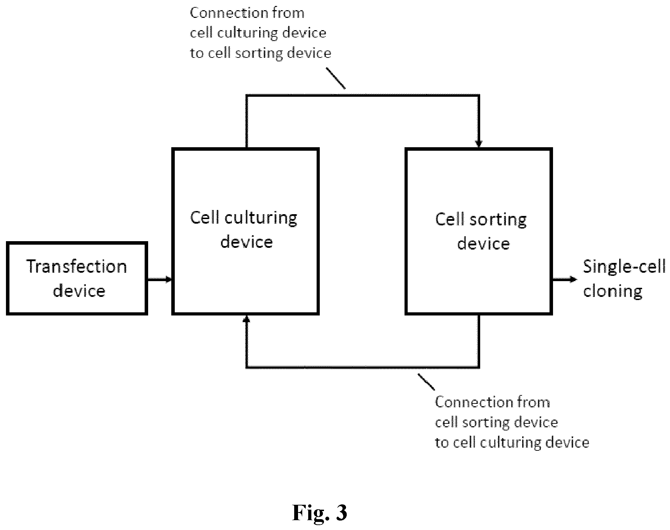

**Fig. 3****.** Schematic illustration of an embodiment of the system according to the present invention, comprising a cell culturing device (left hand side), a cell sorting device (right hand side), a direct connection from the cell culturing device to the cell sorting device (top), and a direct connection from the cell sorting device to the cell culturing device (bottom), and of the method for developing a cell culture process according to the present invention (arrows). The system allows for enriching the cell pool in the cell culturing device with desirable clones, and pre-selecting for growth in the cell culturing device (*e*.*g*., in a bioreactor) by selection in the cell sorting device (*e*.*g*., a FACS). In the embodiment shown, transfection of cells is performed in a separate transfection device, and transfected cells are transferred into the cell culturing device. Following DNA integration in the production cells and cell growth in the cell culturing device *(e.g.,* in a bioreactor), usually over several rounds of cell culturing and sorting, the cells can be directed out of the system for subsequent single cell cloning and clone evaluation.

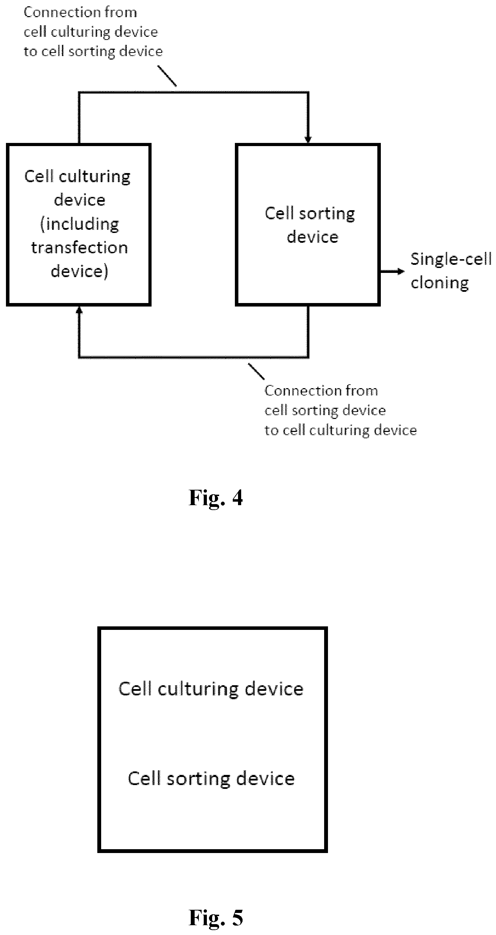

**Fig. 4****.** Schematic illustration of an embodiment of the system according to the present invention, comprising a cell culturing device (left hand side), a cell sorting device (right hand side), a direct connection from the cell culturing device to the cell sorting device (top), and a direct connection from the cell sorting device to the cell culturing device (bottom), and of the method for developing a cell culture process according to the present invention (arrows). The system allows for enriching the cell pool in the cell culturing device with desirable clones, and pre-selecting for growth in the cell culturing device *(e.g.,* in a bioreactor) by selection in the cell sorting device *(e.g.,* a FACS). In the embodiment shown, the transfection device is integrated into the cell culturing device.
Fig. 5. Schematic illustration of an embodiment of the system according to the present invention, wherein the cell culturing device and the cell sorting device are fitted in the same housing or apparatus.

### Detailed Description of the Invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only and is not intended to be limiting. It must be noted that, as used in the specification and the appended claims, the singular forms "a", "an", and "the" include singular and/or plural referents unless the context clearly dictates otherwise. It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, for prior art documents that disclose standard or routine methods. In that case, the incorporation by reference has mainly the purpose to provide sufficient enabling disclosure and avoid lengthy repetitions.

According to a first aspect, the present invention relates to a system comprising at least
(i) a cell culturing device, and
(ii) a cell sorting device.

According to one embodiment, the present invention relates to a system comprising at least
(i) a cell culturing device, and
(ii) a cell sorting device, and
(iii) a direct connection from said cell culturing device to said cell sorting device, and/or
(iv) a direct connection from said cell sorting device to said cell culturing device.

According to one embodiment, the present invention relates to a system comprising at least
(i) a cell culturing device, and
(ii) a cell sorting device, and
(iii) an indirect connection from said cell culturing device to said cell sorting device, and/or
(iv) an indirect connection from said cell sorting device to said cell culturing device.

According to one embodiment, the present invention relates to a system wherein the cell culturing device and the cell sorting device are fitted in the same housing or apparatus.

As used herein, the term "system" refers to a delimitable, natural or artificial structure, which consists of various components with different properties, which are viewed as a common whole due to certain ordered relationships with one another. The components of the system may be arranged in a common housing or apparatus, or the components may be arranged in different housings which are structurally and/or functionally connected to each other.

As used herein, the terms "cell culture" or "culture" refers to the maintenance of cells in an artificial, *in vitro* environment. It is to be understood, however, that the term "cell culture" or "culture" is a generic term and may be used to encompass the maintenance not only of individual cells, but also of tissues, organs, organ systems, for which the terms "tissue culture", "organ culture", "organ system culture" or "organotypic culture" may occasionally be used interchangeably with the term "cell culture", or of unicellular organisms. The cell cultivated in said "cell culture" or "culture" may be any cell which grows in suspension culture, or may be any adherent cell (*i*.*e*., a cell which adheres to a solid surface or support, such as, for example, microbeads to which the cells adhere).

As used herein, the terms "cell culture medium", "culture medium" or "fermentation medium" refer to a solution or suspension containing nutrients used for growing the cells, and shall refer to all kinds of media which are used in the context of culturing cells. Typically, a cell culture medium comprises amino acids, at least one carbohydrate as an energy source, trace elements, vitamins, salts and possibly additional components (e.g. in order to influence cell growth and/or productivity and/or product quality). As used herein, the terms "cell culture medium", "culture medium" or "fermentation medium" refer to serum-free medium or medium supplemented with serum. The medium according to the present invention may either be serum-free or supplemented with 0.5, 1, 2, 3, 4, 5, 10, 15 or 20% of serum.

Said cell culturing device of the system according to the present invention may be any device suitable for maintenance of cells in an artificial, *in vitro* environment, but preferably is selected from the group consisting of a bioreactor, a stir-tank bioreactor, a spinner flask, a shaker flask, a shaker tube, a wave-mixing bioreactor, a petri dish, a multi-well cell culture plate and a microtiter plate.

Said bioreactor may be selected from the group consisting of an Ambr 15 cell culture bioreactor, an Ambr 250 cell culture bioreactor, an univessel bioreactor, a rocked motion bioreactor, a mobius bioreactor (Merck Millipore), a BioFlo bioreactor (Eppendorf), a Xcellerex bioreactor (Cytiva), a Biolector bioreactor (m2p-labs), a Multifors bioreactor (Infors), a DASbox bioreactor (Eppendorf), and a Micro-matrix bioreactor (Applikon).

Said cell culturing device further can be a stainless steel cell culturing device, a glass cell culturing device, a synthetic or plastics cell culturing device, or a disposable cell culturing device.

Said cell sorting device of the system according to the present invention may be any device suitable for analyzing cells directly, or any secreted components from those cells, sorting, selecting and/or separating of cells. Preferably, said cell sorting device can be a fluorescence-activated cell sorter (FACS), a magnetic activated cell sorter (MACS), a microchip-based cell sorting device, or a benchtop flow cytometry cell sorter.

Clone evaluation methods by use of the cell sorting device may employ flow cytometry approaches. For use of flow cytometry to sort and select engineered cell lines that secrete high levels of desired recombinant protein, two approaches have been adopted (for example see WO 2012/001073).

The first or indirect approach, also termed "co-marker expression approach", involves the selection of cells in which a fluorescent co-marker (*e.g*., GFP) or an enzyme such as DHFR (when combined with a fluorescent substrate) is overexpressed. For such selection method to succeed, the expression of the markers must be linked with the expression of the desired protein product. This linkage is required to ensure that cells expressing high levels of the marker also express and secrete high levels of the desired protein product. An example for this approach is described in Kim *et al.* (2012).

The second or direct approach, which is based on identification of membrane-bound or associated protein expression, involves the direct selection of cells that secrete high levels of desired protein product. This direct-selection flow cytometry-based method exploits the observed correlation between membrane bound levels of desired protein product with secretion levels. An example of this approach can be found in Marder P. *et al.* (1990). In this study the authors stained the membrane of hydridoma cells with fluorescently conjugated anti-product antibodies and then sorted and selected the most highly fluorescent cells. They then demonstrated that the resulting sub-clones exhibited enhanced IgG secretion levels in comparison to the cells prior to sorting. Subsequent reports with different cell lines have demonstrated similar results.

A related but more complex alternative to staining and then sorting on membrane levels of product involves the use of product entrapment approaches such as the gel microdrop (GMD) technique or matrix-based secretion assays. In such approaches, secreted antibody is retained by either cross linkage to the cell membrane or within gel microdrops, or immobilised on an artificial matrix on the cell surface prior to FACS sorting with anti-product antibody on levels of fluorescence.

Clone evaluation methods by use of the cell sorting device may also employ alternative single cell assay approaches.

Applebaum *et al.* (US 2010/0028904) described a method to screen and select for highly expressing cells by plating the cells in methylcellulose (semi-solid media) containing fluorescent ProteinA/G to detect product expression.

An automated device for the selection of high-expressing clones used in antibody discovery and cell line development, wherein single clones are immobilised in a semi-solid substrate and then can be screened for product production (ClonePix, Molecular Devices). Further products allow for single cell isolation, growth and assay of product expression, wherein the preferred clone can be retrieved (Beacon, Berkeley Lights; Cyto-Mine, Sphere Fluidics).

As one parameter for clone evaluation methods, relative cell size was described in WO 2012001073). A subpopulation of cells has been found to exist which are larger (more voluminous, as measured by FSC and/or SSC characteristics in flow cytometry) in comparison to the other cells within the cell population. This subpopulation is distinct from the main population, when analysed by, for instance, forward scatter width (FSC-W) or forward scatter area (FSC-A). When cells from this subpopulation are cultured, the clones express more of the polypeptide of interest than the cells of the main cell population, which are smaller in size (as measured by FSC and/or SSC characteristics).

Said cell sorting device according to the present invention may be within the culture environment.

Ultra-high-speed cell sorting devices have been described in the prior art (*e.g*., Leary J.F. 2005; Pereira *et al.* 2018). The adaptation of cell sorting devices to sterile and biosafe working conditions and environment also has been described in the prior art (*e*.*g*., Lennartz *et al.* 2005; Cossarizza *et al.* 2017).

Said direct connection(s) of the system according to the present invention from said cell culturing device to said cell sorting device is suitable for transferring cells from said cell culturing device to said cell sorting device, and said direct connection(s) of the system according to the present invention from said cell sorting device to said cell culturing device is suitable for transferring cells from said cell sorting device to said cell culturing device.

Said direct connection(s) from said cell culturing device to said cell sorting device, and said direct connection(s) from said cell sorting device to said cell culturing device are selected from the group consisting of tubes, lines, flexible hoses, pipes or cables.

Said direct connection(s) of the system according to the present invention may comprise pumps for transferring the cells, preferably wherein the pumps are automatically controlled.

Said indirect connection(s) of the system according to the present invention from said cell culturing device to said cell sorting device is suitable for transferring cells from said cell culturing device to said cell sorting device, and said indirect connection(s) of the system according to the present invention from said cell sorting device to said cell culturing device is suitable for transferring cells from said cell sorting device to said cell culturing device.

Said indirect connection(s) may include, but not be limited to, automated pipetting robot(s) for transferring cells from said cell culturing device to said cell sorting device, and/or for transferring cells from said cell sorting device to said cell culturing device. Such automated pipetting robot(s) may take out culture liquid comprising cells from the culturing device and transfer the culture liquid comprising cells to the cell sorting device, or vice versa. Alternatively, said indirect connection(s) may include, but not be limited to, manual transfer of cells from said cell culturing device to said cell sorting device, and/or manual transfer of cells from said cell sorting device to said cell culturing device.

Said indirect connection(s) may be used together with any of the cell culturing devices described above, and with any of the cell sorting devices as described above.

In a preferred embodiment, said indirect connection(s) may be used with a cell culturing device as described above operating in a batch or fed-batch culture mode and with any cell sorting device as described above. More preferred, said indirect connection(s) may be used with an Ambr cell culture bioreactor operating in a batch or fed-batch culture mode and a cell sorting device. Even more preferred, automated pipetting robot(s) may be used as indirect connection(s) with an Ambr cell culture bioreactor operating in a batch or fed-batch culture mode and with a cell sorting device.

In an embodiment of the present invention, the system according to the present invention may further comprise a device for genetic modification of cells by physical transfection, preferably by at least one of the methods selected from the group consisting of electroporation, sonoporation, mechanoporation, magnetofection, microinjection, gene injection, laserfection, optical transfection and biolistic transfection; or by chemical transfection, preferably by at least one of the methods selected from the group consisting of calcium phosphate-based, cationic lipid-based, DEAE-dextran-based and nano-particle-based transfection; and/or by viral transfection (transduction), preferably by at least one of the methods selected from the group consisting of retroviral, lentiviral, herpes-viral, adenoviral, and adeno-associated viral based transfection (Fig. 3).

Alternatively, genetic modification of cells by physical transfection, chemical transfection, and/or by viral transfection (transduction) may be performed within the cell culturing device. To this end, for example, the cell culturing device according to the present invention, e.g., the bioreactor, is filled with a small volume of culture liquid comprising the cells to be transduced at a suitable cell viability and cell density, and liposomal-based or non-liposomal transfection reagents are added to the cells (chemical transfection, see below).

This system comprising a device for genetic modification of cells (also termed "transfection device") may further comprise a direct connection between said device for genetic modification of cells and the cell culturing device. Said direct connection(s) from said device for genetic modification of cells to the cell culturing device may be selected from the group consisting of tubes, lines, flexible hoses, pipes or cables.

In an embodiment of the present invention, the transfection device is integrated into the cell culturing device (Fig. 4). In one embodiment of the present invention, the walls of the cell culturing device, *e.g.,* the bioreactor, are embedded with the electrodes required for the electroporation process, similarly as with currently used electroporation cuvettes (physical transfection, see below).

In an embodiment of the present invention, the system according to the present invention may further comprise a device to which the cell sorting device can separate out one or more single cell-sorted cells. Preferably, said device is suited for single-cell cloning and/or for expanding the one or more single cell-sorted cells.

This system comprising a device to which the cell sorting device can separate out one or more single cell-sorted cells may comprise a direct connection between the cell sorting device and said device to which the cell sorting device can separate out one or more single cell-sorted cells. Said direct connection(s) from said cell sorting device to said device to which the cell sorting device can separate out one or more single cell-sorted cells may be selected from the group consisting of tubes, lines, flexible hoses, pipes or cables.

Said system according to the present invention further may be a fully closed system and/or a system which is kept sterile inside.

Said system according to the present invention further may be a partially or fully automated system.

As used herein, the term "transfection" relates to any process of deliberately introducing foreign nucleic acids into eukaryotic cells. For transfection into eukaryotic cells various types of nucleic acids into mammalian cells including deoxyribonucleic acids (DNAs), ribonucleic acids (RNAs) as well as small, non-coding RNAs such as siRNA, shRNA, and miRNA can be employed. Transfection can be classified into two types, namely stable and transient transfection. Stable transfection refers to sustaining long-term expression of a transgene by integrating the foreign nucleic acid into the host nuclear genome, whereas transient transfection does not require integrating nucleic acids into the host cell genome (Fus-Kujawa *et al.* 2021).

The choice of the optimal transfection method depends on factors including the type and origin of cells and the form of introduced nucleic acids. For introduction of foreign (modified homologous and/or heterologous) nucleic acid(s), encoding the gene(s) of interest, into eukaryotic cells in the transfection device or cell culturing device, for example bioreactor, according to the present invention, physical transfection, chemical transfection, or viral vector-based transfection (transduction) may be employed.

For physical transfection by electroporation, high-voltage electric shocks are applied for transiently increasing the cell membrane permeability and thus allowing entry of different kinds of molecules into the cells. The external electric field, which can outperform the cell membrane capacitance, can induce temporary but reversible disruption in cell membrane permeability. This temporary state of the membrane allows translocation of different molecules into the cell. The translocation occurs by either simple diffusion or electrophoretical passage through the cell membrane (Fus-Kujawa *et al.* 2021, Chong *et al.* 2021).

Sonoporation or ultrasound-assisted transfection involves the use of microbubbles to create temporary holes on the cell membrane to ease the transfer of genetic materials. Laser irradiation-assisted transfection uses a laser beam to create small holes on the plasma membrane to allow entry of foreign genetic substances. The mechanism, by which molecules enter into cells during laserfection still remains unclear; however, it appears that either the laser light creates small transient holes in the cell membrane or a shock wave, produced by the laser beam absorption in the medium, induces a mechanical stress, which affects cellular membrane. Like electroporation, both sonoporation and laser-assisted transfection also pose some risk of damaging the cell membrane and irreversible cell death; hence, transfection conditions have to be well adjusted to the respective cell type.

Magnetofection or magnet-assisted transfection, that uses magnetic force to aid in the transfer of foreign genetic materials, has been described to be less destructive to the host cell, but to have lower efficiency (Chong *et al.* 2021).

Microinjection is a common technique that has been applied to study a variety of experimental issues in many types of cells. It is uniquely effective in transfecting cells that are difficult in processing with other methods, *e.g*., mesenchymal stem cells or smooth muscle cells. Transfection efficiency of single-cell microinjection is very high, however, its application to larger numbers of cells is less feasible.

Biolistic transfection is a mechanical method which has potential applications in wide variety of cell and tissue types. Initially developed for transfection of plant cells, biolistic transfection is based on DNA or RNA being immobilized to sub-cellular size particles. Those particles are then accelerated to high velocity using a "gene gun" and then shot into cells. Biolistic method can be used for both *in vivo* and *in vitro* cell transfections, hence it has been widely applied also for use in gene therapy. The method allows for transfecting large quantities of cells per single use, and for possible co-transfection of two or more DNAs in a single use, making it time-efficient and simple to use. Nano-biolistic method is employing particles with smaller size of about 40 nm (Fus-Kujawa *et al.* 2021; Chong *et al.* 2021).

Chemical transfection can be categorized into non-liposomal-based and liposomal-based methods. Non-liposomal transfection reagents can be further divided into several classes, including calcium phosphate, dendrimers, polymers, nanoparticles and non-liposomal lipids.

Calcium phosphate is one of the cheapest chemicals used in transfection that involves binding of the positively charged calcium ions (Ca2⁺) with the negatively charged nucleic acids to form a precipitate before being taken up by host cells. However, the success rate of calcium phosphate transfection is comparatively low and requires prior optimization to achieve high transfection efficiency. Endocytosis or phagocytosis are the mechanisms by which calcium phosphate as transfection reagent mediates uptake of the nucleic acid.

Dendrimers are three-dimensional, highly branched organic macromolecules that can form complexes with nucleic acids which have been described to be superior to calcium phosphate as an alternative non-liposomal transfection reagent. However, transfection efficiency using dendrimers is still lower than viral vectors and liposomal reagents.

Cationic polymers can also form complexes with the negatively charged nucleic acids, which aid in the uptake of the genetic materials by cells through endocytosis. Compared to viral vectors and lipofection, cationic polymers produce less cytotoxicity, but are compromised with lower efficiency.

Diethylaminoethyl-dextran (DEAE-dextran) is a cationic polymer that forms complexes with negatively charged DNA. It is used as a complexing agent for nucleic acids (DNA and RNA) and also as a coating of liposomes. Complexation of DNA with DEAE-dextran via electrostatic interactions results in positively charged complex formation that adheres with negatively charged plasma membrane. Entering of this complex into the cytoplasm is possible through osmotic shock induced by dimethyl sulfoxide (DMSO) or glycerol, or it crosses the plasma membrane via endocytosis. Despite this method is inexpensive and easy to perform, applied high concentration of DEAE-dextran can be toxic for transfected/treated cells. So far, DEAE-dextran has been successfully used only for transient transfection, and its efficiency varies between cell types; the efficiency is usually less than 10% in primary cells.

Recently, polymeric nanoparticles have been emerging as an alternative option in non-liposomal transfection due to their small size, which enhances the entry of nucleic acids into the host cell. Nanoparticles were reported to cause little cytotoxicity to the transfected cells. A variety of natural and synthetic polymers have been used in order to effect an efficient delivery of nucleic acids, such as, for example, folic acid (FA)-conjugated hydroxypropyl-chitosan (HPCS) nanoparticles for the efficient and targeted delivery of antisense oligonucleotides.

Non-liposomal lipid-based nanocarrier (nanoparticles made of solid lipids) is a type of lipid-formulated nanocarrier that allows fast and effective delivery of nucleic acids into eukaryotic cells. Compared to liposomal lipid nanocarriers, this nanocarrier was described to be safer, but its preparation is tedious and expensive.

Liposomal-based transfection reagents are chemicals that enable the formation of positively charged lipid aggregates that could merge smoothly with the phospholipid bilayer of the host cell to allow the entry of the foreign genetic materials with minimal resistance (Chong *et al.* 2021; Fajrial *et al.* 2020).

Viral vector-based transfection, termed "transduction", involves using a viral vector to carry a specific nucleic acid sequence into a host cell. Retroviruses, such as Lentiviruses, are often used for stable transfection; in contrast, adenovirus, adeno-associated virus (AAV) and herpes virus are viral vectors which do not guarantee stable transfection. As compared to non-viral transfection, viral transduction is widely recognized as a highly effective method to transfect difficult-to-transfect cells such as primary cells. Generally, retroviruses can only be used to transduce dividing cells, while adenoviruses, AAVs and herpes viruses can be used to transduce both dividing and non-dividing cells.

Unlike the genomes of adenoviruses, AAVs and herpes viruses which are maintained episomally, a retroviral genome is integrated into the host genome. Integrase is a type of enzyme produced by retroviruses that facilitate the integration of foreign genetic materials into the host genome. In retroviruses, RNAs will be reverse transcribed into a doublestranded viral DNA before being integrated into the host cell genome for replication and expression (Chong *et al.* 2021).

According to a second aspect, the present invention relates to a method for developing and/or optimizing a cell culture process, comprising at least the steps of
(a) growing a cell culture of a polyclonal population of cells in a cell culturing device,
(b) selecting cells in a cell sorting device,
(c) optionally repeating said steps (a) to (b) at least once.

According to an embodiment, the present invention relates to a method for developing and/or optimizing a cell culture process, comprising at least the steps of
(a) growing a cell culture of a polyclonal population of cells in a cell culturing device,
(b) transferring cells from the cell culturing device into a cell sorting device by direct connection(s) from the cell culturing device to the cell sorting device,
(c) selecting cells in the cell sorting device,
(d) recirculating selected cells from the cell sorting device back to the cell culturing device by direct connection(s) from the cell sorting device to the cell culturing device,
(e) optionally repeating said steps (a) to (d) at least once.

According to an embodiment, the present invention relates to said method for developing and/or optimizing a cell culture process, comprising prior to step (a) a further step of genetically modifying cells by
physical transfection, preferably by at least one of the methods selected from the group consisting of electroporation, sonoporation, magnetofection, microinjection, gene injection, laserfection, optical transfection and biolistic transfection, and/or by
chemical transfection, preferably by at least one of the methods selected from the group consisting of calcium phosphate-based, cationic lipid-based, DEAE-dextran-based and nano-particle-based transfection, and/or by
viral transfection (transduction), preferably by at least one of the methods selected from the group consisting of retroviral, lentiviral, herpes-viral, adenoviral, and adeno-associated viral based transfection.

In an embodiment of the present invention, in said method for developing and/or optimizing a cell culture process comprising prior to step (a) a further step of genetically modifying cells by physical transfection, chemical transfection and/or viral transfection (transduction), this step of genetically modifying cells is performed in the device for genetic modification of cells as described above, or in the cell culturing device of the system as described above.

In an embodiment of the present invention, said cell culture process may be suited for upscaling to large-scale manufacturing, and/or said cell culturing device may be a small-scale cell culturing device.

Said cell culture process according to the present invention can be a batch culture, a fed-batch culture, a continuous culture, and/or a perfusion culture process.

As used herein, the term "batch culture" refers to a cell culture process wherein a cell culture unit or device is filled with a defined working volume of cell culture medium, which is not exchanged during the culturing period, and inoculated with cells; the culture usually runs until the nutrients are exhausted or the waste products reach toxic levels and the cells stop growing.

As used herein, the term "fed-batch culture" refers to a cell culture process which is similar to batch-mode, but the initial volume of medium only fills a portion of the cell culture unit or device and when the nutrients are depleting, feed medium of the same or different composition is added to the cell culture unit or device to replenish nutrients, until a maximum working volume is reached.

As used herein, the term "perfusion culture" or "perfusion mode" refers to a cell culture process wherein the use of a cell retention device inside or outside of the cell culture unit or device allows spent medium to be removed from the cell culture unit or device without removing the cells, while adding the same amount of fresh medium back to the unit or device thus achieving a constant culture volume in the unit or device.

As used herein, the term "continuous culture" or "continuous mode" refers to a cell culture process wherein a mixture of cells and medium in the cell culture unit or device is continuously removed as fresh medium is added to the unit or device. The rate of removal is determined by the growth rate of the cells and must be carefully controlled in order to avoid washout. In this mode, ideally cells can be kept growing in the exponential phase while maintaining a constant cell density and volume in the culture unit or device.

In an embodiment of the present invention, in said cell culture process prior to step (a) the cells have been genetically modified by transfection, transduction and/or gene editing tools to comprise at least one modified homologous and/or at least one heterologous polynucleotide, preferably wherein said polynucleotide has been stably integrated in the genome of the cells, and/or preferably wherein said genetic modification is performed within said cell culturing device.

In an embodiment of the present invention, in said cell culture process, after performing steps (a) to (d) at least once, the cells selected in the cell sorting device in step (c) are subjected to single-cell cloning.

In said cell culture process according to the present invention, the cell culturing device may be any device suitable for maintenance of cells in an artificial, *in vitro* environment, but preferably is selected from the group consisting of a bioreactor, a stir-tank bioreactor, a spinner flask, a shaker flask, a shaker tube, a wave-mixing bioreactor, a petri dish, a multi-well cell culture plate and a microtiter plate. Said culturing device may be an Ambr 15 bioreactor.

Said bioreactor may be selected from the group consisting of an Ambr 15 cell culture bioreactor, an Ambr 250 cell culture bioreactor, an univessel bioreactor, a rocked motion bioreactor, a mobius bioreactor (Merck Millipore), a BioFlo bioreactor (Eppendorf), a Xcellerex bioreactor (Cytiva), a Biolector bioreactor (m2p-labs), a Multifors bioreactor (Infors), a DASbox bioreactor (Eppendorf), and a Micro-matrix bioreactor (Applikon).

In said cell culture process according to the present invention, the cell culturing device further can be a stainless steel cell culturing device, a synthetic or plastics cell culturing device, or a disposable cell culturing device.

In said cell culture process according to the present invention, the cell sorting device may be any device suitable for sorting, selecting and/or separating of cells. Preferably, said cell sorting device can be a fluorescence-activated cell sorter (FACS), a magnetic activated cell sorter (MACS), a microchip-based cell sorting device, or a benchtop flow cytometry cell sorter.

In said cell culture process according to the present invention, the direct connection(s) from the cell culturing device to the cell sorting device is suitable for transferring cells from the cell culturing device to the cell sorting device, and said direct connection(s) from the cell sorting device to the cell culturing device is suitable for transferring cells from the cell sorting device to the cell culturing device.

In said cell culture process according to the present invention, the direct connection(s) from the cell culturing device to the cell sorting device, and the direct connection(s) from the cell sorting device to the cell culturing device are selected from the group consisting of tubes, lines, flexible hoses, pipes or cables.

In said cell culture process according to the present invention, further the direct connection(s) may comprise pumps for transferring the cells, preferably wherein the pumps are automatically controlled.

In the system according to the present invention, or in the method for developing and/or optimizing a cell culture process according to the present invention, said cell is selected from an animal cell, a mammalian cell, an insect cell, a plant cell, an algae cell and a fungus cell. Preferably, said cell is a mammalian cell, further preferably said cell is an immortalized cell line or a stem cell, more preferably said cell is a human, simian or rodent cell line, most preferably said cell line is selected from the group consisting of Chinese Hamster Ovary (CHO) cells, baby hamster kidney (BHK) cells, COS cells, mouse myeloma cells (NS0, SP2/0), human embryonic kidney (HEK) cells, human retina-derived cells (PER-C6), and human amniocyte cells (CAP cells).

BHK21 ("Baby Hamster Kidney") cells belong to a quasi-diploid established line of Syrian hamster cells, descended from a clone from an unusually rapidly growing primary culture of newborn hamster kidney tissue. Non limiting examples for BHK-21 cell lines which are commercially available and can be used in the context of the present invention are BHK-21 (C-13); BHK21-pcDNA3.1-HC; BHK570; Flp-In-BHK Cell Line; and/or BHK 21 (Clone 13) hamster cell line.

Chinese hamster ovary (CHO) cells are a cell line derived from the ovary of the Chinese hamster. Introduced in the 1960s and originally grown as a monolayer culture, they are often used in biological and medical research and commercially in the production of therapeutic proteins. CHO cells are still the most commonly used mammalian hosts for industrial production of recombinant protein therapeutics and are usually grown in suspension culture.

Non limiting examples for CHO cell lines which are commercially available and can be used in the context of the present invention are FreeStyle CHO-S cells; ER-CHO Cell Line; CHO 1-15 500 CHINESE HAM; CHO-DXB, CHO-dhfr-, CHO DP-12 clone#1934; CHO-CD36; CHO-ICAM-1; CHO-K1; Ovary; HuZP3-CHOLec3.2.8.1; xrs5; CHO-K1/BB2 Cells; CHO-K1/BB3 Cells; CHO-K1/EDG8/Galpha15 Cells; CHO-K1/M5 Cells; CHO-K1/NK1 Cells; CHO-K1/NK3 Cells; CHO-K1/NMUR1 Cells; CHO-K1/NTSR1 Cells; CHO-K1/OX1 Cells; CHO-K1/PAC1/Gα15 Cells; CHO-K1/PTAFR Cells; CHO-K1/TRH1 Cells; CHO-K1/V1B Cells; 5HT1A Galpha-15-NFAT-BLA CHO-K1 Cell Line; AVPR2 CRE-BLA CHO-K1 Cell Line; CHO-S Cells SFM Adapted; DG44 Cells; Flp-In-CHO Cell Line; GeneSwitch-CHO Cell Line; NFAT-bla CHO-K1 Cell Line; T-REx-CHO Cell Line; GenoStat CHO K-1 Stable Cell Line; GenoStat CHO K-1 Stable Cell Line Kit; CHO-K1 Cell Line hamster, CHO-PEPT1 Cell line. In a particularly preferred embodiment, the hamster cell-based expression system is a CHO-dhfr⁻-cell line.

In said method for developing and/or optimizing a cell culture process according to the present invention, said cell is subjected to a transfection method selected from the group consisting of physical transfection methods, preferably wherein physical transfection methods are electroporation, sonoporation, magnetofection, and biolistic transfection; chemical transfection methods, preferably wherein chemical transfection methods are calcium phosphate-based, cationic lipid-based, DEAE-dextran-based and nano-particle-based transfection; and/or viral transfection (transduction) methods, preferably wherein viral transfection methods are based on retroviral, lentiviral, adenoviral, and adeno-associated viral transfection. Said transfection methods have been further described above.

In said method for developing and/or optimizing a cell culture process according to the present invention, the culturing conditions for which the cell culture process is to be optimized may include but not be limited to culture medium, culture temperature, pH value, oxygen concentration, cell density, addition of cell nutrients including the time of addition and the target concentration of the nutrients in the culture medium, culturing time, specific power input, rate of gas supply, rate of perfusion, and osmolarity of the culture medium.

In said method for developing and/or optimizing a cell culture process according to the present invention, further said selection of cells in the cell sorting device is performed according to target parameters may include but not be limited to high cell viability, cell size, high and/or stable cell density, high and/or stable yield of a biomolecule of interest produced by the cells in the cell culture, and a desired property of a biomolecule of interest produced by the cells in the cell culture.

According to a third aspect, the present invention relates to the use of the system according to the present invention in a method for developing and/or optimizing a cell culture process according to the present invention.

In said method for developing and/or optimizing a cell culture process according to the present invention, or with regard to said use of the system according to the present invention, said cell culture process is suited for producing biopharmaceuticals, such as recombinant polypeptides, proteins, vaccines, and/or cellular biopharmaceuticals. Preferably, said proteins are selected from the group consisting of glycoproteins, antibodies, cytokines, receptors, fusion proteins, and viral proteins, or fragments thereof.

Said "biopharmaceutical", also termed "biologic", "biological drug" or "biologic therapeutic" according to the present invention preferably is an antibody, or antigen-binding fragment thereof, or antigen-binding derivative thereof, or antibody-like protein, or an aptamer.

As used herein, the term "antibody" shall refer to a protein consisting of one or more polypeptide chains encoded by immunoglobulin genes or fragments of immunoglobulin genes or cDNAs derived from the same. Said immunoglobulin genes include the light chain kappa, lambda and heavy chain alpha, delta, epsilon, gamma and mu constant region genes as well as any of the many different variable region genes.

The basic immunoglobulin (antibody) structural unit is usually a tetramer composed of two identical pairs of polypeptide chains, the light chains (L, having a molecular weight of about 25 kDa) and the heavy chains (H, having a molecular weight of about 50-70 kDa). Each heavy chain is comprised of a heavy chain variable region (abbreviated as VH or V_{H}) and a heavy chain constant region (abbreviated as CH or C_{H}). The heavy chain constant region is comprised of three domains, namely CH1, CH2 and CH3. Each light chain contains a light chain variable region (abbreviated as VL or V_{L}) and a light chain constant region (abbreviated as CL or C_{L}). The VH and VL regions can be further subdivided into regions of hypervariability, which are also called complementarity determining regions (CDR) interspersed with regions that are more conserved called framework regions (FR). Each VH and VL region is composed of three CDRs and four FRs arranged from the amino terminus to the carboxy terminus in the order of FR1, CDR1, FR2, CDR2, FR3, CDR3, FR4. The variable regions of the heavy and light chains form a binding domain that interacts with an antigen.

The CDRs are most important for binding of the antibody or the antigen binding portion thereof. The FRs can be replaced by other sequences, provided the three-dimensional structure which is required for binding of the antigen is retained. Structural changes of the construct most often lead to a loss of sufficient binding to the antigen.

The term "antigen binding portion" of the (monoclonal) antibody refers to one or more fragments of an antibody which retain the ability to specifically bind to the antigen in its native form. Examples of antigen binding portions of the antibody include a Fab fragment, a monovalent fragment consisting of the VL, VH, CL and CH1 domains, an F(ab')₂ fragment, a bivalent fragment comprising two Fab fragments linked by a disulfid bridge at the hinge region, an Fd fragment consisting of the VH and CH1 domain, an Fv fragment consisting of the VL and VH domains of a single arm of an antibody, and a dAb fragment which consists of a VH domain and an isolated complementarity determining region (CDR).

The antibody, or antibody fragment or antibody derivative thereof, according to the present invention can be a monoclonal antibody. The antibody can be of the IgA, IgD, IgE, IgG or IgM isotype.

As used herein, the term "monoclonal antibody (mAb)" shall refer to an antibody composition having a homogenous antibody population, *i.e.,* a homogeneous population consisting of a whole immunoglobulin, or a fragment or derivative thereof, derived from a monoclonal antibody-producing cell, or synthetically produced. Particularly preferred, such antibody is selected from the group consisting of IgG, IgD, IgE, IgA and/or IgM, or a fragment or derivative thereof.

As used herein, the term "fragment" shall refer to fragments of such antibody retaining target binding capacities, *e.g*., a CDR (complementarity determining region), a hypervariable region, a variable domain (Fv), an IgG heavy chain (consisting of VH, CH1, hinge, CH2 and CH3 regions), an IgG light chain (consisting of VL and CL regions), and/or a Fab and/or F(ab)₂.

As used herein, the term "derivative" shall refer to protein constructs being structurally different from, but still having some structural relationship to, the common antibody concept, *e.g.,* scFv, Fab and/or F(ab)₂, as well as bi-, tri- or higher specific antibody constructs. These items are further explained below.

Other antibody derivatives known to the skilled person are Diabodies, Camelid Antibodies, Domain Antibodies, bivalent homodimers with two chains consisting of scFvs, IgAs (two IgG structures joined by a J chain and a secretory component), shark antibodies, antibodies consisting of new world primate framework plus non-new world primate CDR, dimerised constructs comprising CH3+VL+VH, other scaffold protein formats comprising CDRs, and antibody conjugates.

As used herein, the term "antibody-like protein" refers to a protein that has been engineered (*e.g.* by mutagenesis of Ig loops) to specifically bind to a target molecule. Typically, such an antibody-like protein comprises at least one variable peptide loop attached at both ends to a protein scaffold. This double structural constraint greatly increases the binding affinity of the antibody-like protein to levels comparable to that of an antibody. The length of the variable peptide loop typically consists of 10 to 20 amino acids. The scaffold protein may be any protein having good solubility properties. Preferably, the scaffold protein is a small globular protein. Antibody-like proteins include without limitation affibodies, anticalins, and designed ankyrin proteins, and affilin proteins. Antibody-like proteins can be derived from large libraries of mutants, *e.g*. by panning from large phage display libraries, and can be isolated in analogy to regular antibodies. Also, antibody-like binding proteins can be obtained by combinatorial mutagenesis of surface-exposed residues in globular proteins.

As used herein, the term "Fab" relates to an IgG fragment comprising the antigen binding region, said fragment being composed of one constant and one variable domain from each heavy and light chain of the antibody.

As used herein, the term "F(ab)₂" relates to an IgG fragment consisting of two Fab fragments connected to one another by disulfide bonds.

As used herein, the term "scFv" relates to a single-chain variable fragment being a fusion of the variable regions of the heavy and light chains of immunoglobulins, linked together with a short linker, usually comprising serine (S) and/or glycine (G) residues. This chimeric molecule retains the specificity of the original immunoglobulin, despite removal of the constant regions and the introduction of a linker peptide.

Modified antibody formats are for example bi- or trispecific antibody constructs, antibody-based fusion proteins, immunoconjugates and the like.

IgG, scFv, Fab and/or F(ab)₂ are antibody formats which are well known to the skilled person. Related enabling techniques are available from respective textbooks.

According to preferred embodiments of the present invention, said antibody, or antigen-binding fragment thereof or antigen-binding derivative thereof, is a murine, a chimeric, a humanized or a human antibody, or antigen-binding fragment or antigen-binding derivative thereof, respectively.

Monoclonal antibodies (mAb) derived from mouse may cause unwanted immunological side-effects due to the fact that they contain a protein from another species which may elicit antibodies. In order to overcome this problem, antibody humanization and maturation methods have been designed to generate antibody molecules with minimal immunogenicity when applied to humans, while ideally still retaining specificity and affinity of the non-human parental antibody. Using these methods, *e.g*., the framework regions of a mouse mAb are replaced by corresponding human framework regions (so-called CDR grafting).

As used herein, the term "humanized antibody" relates to an antibody, a fragment or a derivative thereof, in which at least a portion of the constant regions and/or the framework regions, and optionally a portion of CDR regions, of the antibody is derived from or adjusted to human immunoglobulin sequences.

Said "cellular biopharmaceuticals" produced by the cell culture process according to the present invention are preferably selected from the group consisting of pluripotent stem cells (PSCs), including embryonic stem cells (ESCs), epiblast stem cells (EpiSCs), embryonic germ cells (EGCs), and induced pluripotent stem cells (iPSCs); adult stem cells (ASCs), including hematopoietic stem cells (HSCs), skin stem cells (SSCs), neural stem cells (NSCs), and mesenchymal stem cells (MSCs); and cancer stem cells (CSCs); non-stem cell-based cells and modified immune cells including tumor-infiltrating lymphocytes (TILs), tumor-specific T-cell receptor (TCR)-modified T cells, chimeric antigen receptor (CAR)-T cells, CAR-NK cells, lymphokine activated killer (LAK) cells, cytokine-induced killer (CIK) cells, γδ-T cells, and NK cells.

As used herein, the term "cell therapy" refers to the transfer of autologous or allogeneic cellular material into a patient for medical purposes. Cell therapy encompasses stem cell- and non-stem cell-based unicellular or multicellular therapies. It typically employs autologous or allogeneic cells; it might involve genetic engineering of the cells, and can be administered topically or as injectables, infusions, bioscaffolds, or scaffold-free systems. Cell therapy approaches span multiple therapeutic areas, such as regenerative medicine, immunotherapy, and cancer therapy (El-Kadiry *et al.* 2021).

Embryonic and adult stem cells are a type of unspecialized, self-renewable cells with varying developmental potency. Developmental potency represents a differentiation continuum ranging from totipotency (*i*.*e*., highest differentiation potential; *e.g.,* zygote) to pluripotency (*e*.*g*., embryonic stem cells), multipotency (*e*.*g*., hematopoietic stem cells), oligopotency (*e*.*g*., myeloid stem cells), and ultimately unipotency (*i.e*., least differentiation potential; *e.g.,* dermatocytes). This hierarchy can be artificially manipulated or reversed by nuclear reprogramming methods, including the use of transcriptional factors, which can eventually induce pluripotency in many cell types. Stem cell specialization is influenced by external signals (*e*.*g*., physical contact between cells, paracrine secretions of nearby tissue, and tissue type), internal signals (*e.g*., genes), and epigenetics (embryonic cell origin).

PSCs give rise to all cell types except extraembryonic placental cells; they include embryonic stem cells (ESCs), found in the inner blastocyst cell mass of preimplantation embryos; epiblast stem cells (EpiSCs), embryonic germ cells (EGCs), found in postimplantation embryos, and induced pluripotent stem cells (iPSCs), derived from direct reprogramming of postnatal/adult somatic cells in vitro.

Somatic or ASCs are rare, undifferentiated cells distributed among differentiated or specialized cells in organs of a developed organism. With more limited self-renewal and differentiation potentials than PSCs, ASCs replenish lost cells or contribute to the healing or growth of cells by giving rise to precursor or progenitor cells and ultimately differentiated cells. ASCs include hematopoietic stem cells (HSCs), skin stem cells (SSCs), neural stem cells (NSCs), and mesenchymal stem cells (MSCs). HSCs are mostly found in the bone marrow and give rise to all mature blood cells, red blood cells, white blood cells, and platelets. SSCs, such as epidermal stem cells and hair follicle stem cells, maintain skin integrity. NSCs are self-renewable stem cells found in the central nervous system and can give rise to nerve cells, oligodendrocytes, and astrocytes. MSCs are of mesodermal, non-hematopoietic origins and are present in multiple tissues, including bone marrow, adipose tissue, peripheral blood, and placenta. They can differentiate into bone, cartilage, and fat cells, as well as cells of ectodermal or endodermal parentage.

CSCs, or tumor-initiating cells, are found within solid and blood tumors and originate from normal stem cells or progenitor cells by several proposed mechanisms, such as mutations, gene transfer, epigenetic alterations, and microenvironmental factors. CSCs possess self-renewal, differentiation, metastasis, and immunosuppressive properties and play an important role in cancer growth, metastasis, relapse, and resistance to chemotherapy and radiotherapy. CSCs have been used for targeting of different signaling pathway-interfering agents that subsequently prevent cancer growth and relapse.

Non-stem cell-based cell therapies are generally somatic cells that are isolated from the human body, propagated, expanded, selected, and subsequently administered to patients for curative, preventive, or diagnostic purposes. Non-stem cell-based cell therapies include fibroblasts, chondrocytes, keratinocytes, hepatocytes, pancreatic islet cells, and immune cells, such as T cells, dendritic cells (DCs), natural killer (NK) cells, and macrophages.

Somatic cell-based therapies are generally employed as an *in vivo* source of enzymes, cytokines, and growth factors; as an adoptive cell therapy (ACT) to treat cancers; as transplanted cells, such as hepatocytes or pancreatic islet cells, to correct inborn metabolic errors; or as scaffold-based or -free cellular systems to treat ulcers, burns, or cartilage lesions.

ACT involves the intravenous transfer of modified peripheral or tumor-resident immune cells into patients to mount an immunologic reaction against tumors. Modified immune cells used in ACT include tumor-infiltrating lymphocytes (TILs), tumor-specific T-cell receptor (TCR)-modified T cells, and chimeric antigen receptor (CAR)-T cells.

CAR-T cells employ synthetic antibody-based chimeric antigen receptors (CARs); the transfer of CARs to T cells can be performed by various techniques, including retroviral transduction. The genetic construct of CARs encodes the single-chain variable fragment (scFv) of a monoclonal antibody (serves as the extracellular antigen recognition domain), a CD3ζ chain (serves as the intracellular signaling domain of the TCR), and a co-receptor, such as CD28, for co-stimulation.

T cells isolated from peripheral blood by leukapheresis can be genetically engineered in vitro to express modified TCRs that can be directed against specific tumor antigens, such as melanoma differentiation antigens and cancer/testis antigens.

Other ACT strategies include lymphokine-activated killer (LAK) cells, cytokine-induced killer (CIK) cells, γδ T cells, and NK cells (El-Kadiry *et al.* 2021).

Said biopharmaceuticals, such as recombinant polypeptides, proteins, vaccines, and/or cellular biopharmaceuticals, produced by use of the system according to the present invention in a method for developing and/or optimizing a cell culture process according to the present invention, can serve for therapeutic and/or preventive purposes as active pharmaceutical ingredients in pharmaceutical compositions, optionally together with one or more pharmaceutically acceptable excipients. Preferably, said excipients can be selected from the group consisting of pharmaceutically acceptable buffers, surfactants, diluents, carriers, excipients, fillers, binders, lubricants, glidants, disintegrants, adsorbents, and/or preservatives.

According to a fourth aspect, the present invention relates to a system comprising more than one of the systems according to the present invention, wherein said system is suitable for developing and/or optimizing a particular polyclonal cell culture with regard to multiple target cell culture parameters and conditions in parallel, and/or for developing and/or optimizing different polyclonal cell cultures with regard to a particular target cell culture parameter or condition, preferably wherein said system comprises a multitude of the systems according to the present invention.

### Example 1

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

For transduction with a viral transgene vector (*e*.*g*., for expression of an antibody), a suitable host cell line (*e*.*g*., CHO DG44 cells) is seeded in the cell culturing device (*e*.*g*., 100 - 250 ml smale-scale bioreactor) of the system according to the present invention at a suitable cell density and grown under cultivation conditions (*e.g*., temperature, pH, stirring speed, feeding conditions) according to a large-scale manufacturing process. The cells are transduced by addition of the viral transgene vector at a suitable multiplicity of infection (M.O.I., *e.g.,* an M.O.I. of 10) and a suitable time period. Medium exchange may occur prior to, and after, viral vector addition to increase genetic modification success of the host line.

Either constantly or at a steady flow rate, cells are transferred via the direct connection from the bioreactor to the cell sorting device, wherein the cell sorting device is a fluorescence-activated cell sorter (FACS). In parallel, cell and medium samples are taken from the cell culturing device (bioreactor) on a reglar basis to assess average cell density, cell growth rate and viability, as well as concentrations and quality of the product expressed by the transduced cells.

Separation of desired cell populations from less desired cell populations is performed in the cell sorting device on the basis of cell morphology (*e.g*., cell size) or on the basis of anti-IgG cell staining. Via the direct connection between the cell sorting device (FACS) and the cell culturing device (bioreactor), desired cell populations are re-circulated back into the bioreactor. The flow rate of cell culture volume circulating through the system of cell culturing device and cell sorting device (closed bioreactor/FACS system) depend uponand are adapted to the average cell growth rate in the bioreactor.

As soon as a pre-determined level of cell performance, including product yield and quality (stability, glycosylation profile etc.), in the bioreactor has been reached, high-producer cells in the polyclonal cell pool, instead of being recirculated to the bioreactor, are directed from the cell sorting device to the device that is suited for single-cell cloning and/or for expanding the single cell-sorted cells via a respective direct connection.

The monoclonal antibody produced by a respective clone is quantified by ELISA assay as described in the prior art. Genomic DNA, total RNA, and cDNA is being extracted from the best cell clone candidates for analysis of transgene copy numbers and mRNA levels, which data are instrumental in final producer cell clone selection.

### Example 2

For liposomal-based transfection of CHO DG44 cells, the cells are passaged at ca. 3×10⁵ cells/ml in CD-DG44 medium (8 mM L-glutamine, 0.18% Pluronic F-68), a chemically defined, protein-free medium for growth and expression of DG44 cells in suspension, in shaker flasks at 130 rpm (37°C, 8% CO₂). About 1.5×10⁷ cells with viability of at least 95% are placed in a total volume of 30 ml CD-DG44 medium in 125-ml shaker flasks. Linearized DNA, comprising an expression cassette encoding heavy and lights chains of a monoclonal antibody, and FreeStyle MAX lipid-based transfection reagent are combined according to instructions from the manufacturer, and incubated for 10-20 min at room temperature. The DNA-lipid mixture is then added to the cells while slowly swirling the shaker flask. After incubation for two days at 130 rpm (37°C, 8% CO₂), the medium is exchanged by selection medium (CD OptiCHO medium; neomycin (G418) selection).

From the transfection device (device for genetic modification of cells), cells are transferred via the direct connection between the transfection device and the cell culturing device to the latter, wherein the cell culturing device is a bioreactor which is equipped for perfusion culture process and has a working volume between 0.5 and 10 liters in total.

Initiating from two days after transfer into the bioreactor, cells are started to be transferred via the direct connection from the bioreactor to the cell sorting device, wherein the cell sorting device is a fluorescence-activated cell sorter (FACS), either constantly at a steady flow rate, or in regular intervals. In parallel, cell and medium samples are taken from the cell culturing device (bioreactor) on a reglar basis to assess average cell density, cell growth rate and viability, as well as concentrations of the product expressed by the transfected cells.

Separation of high-producer cells from low-performing cell clones is performed in the cell sorting device, *e.g.,* on the basis of direct-selection flow cytometry-based method. Via the direct connection between the cell sorting device (FACS) and the cell culturing device (bioreactor), higher-producer cells are re-circulated back into the bioreactor. The flow rate of cell culture volume circulating through the system of cell culturing device and cell sorting device (closed bioreactor/FACS system) and the control of gradual increase in overall culture volume in the bioreactor depend from and are adapted to the average cell growth rate in the bioreactor.

As soon as a pre-determined level of cell performance, including product yield and quality (stability, glycosylation profile etc.), in the bioreactor has been reached, high-producer cells in the polyclonal cell pool, instead of being recirculated to the bioreactor, are directed from the cell sorting device to the device that is suited for single-cell cloning and/or for expanding the single cell-sorted cells via a respective direct connection.

The monoclonal antibody produced by a respective clone is quantified by ELISA assay as described in the prior art. Genomic DNA, total RNA, and cDNA is being extracted from the best cell clone candidates for analysis of transgene copy numbers and mRNA levels, which data are instrumental in final producer cell clone selection.

### References:

Al-Rubeai M. and Emery A.N. 1993. Flow cytometry in animal cell culture. Nat Biotech Vol. 11 No. 5, p. 572-579.
Bleckwenn N.A. and Shiloach J. 2004. Large scale cell culture. In: Current Protocols in Immunology, A.1U.1-A.1U.44, John Wiley & Sons, Inc.
Chong Z.X., Yeap S.K., and Ho W.Y. 2021. Transfection types, methods and strategies: a technical review. PeerJ. Vol. 9:e11165, doi 10.7717/peerj.11165.
Clift M.J.D., Fytianos K., Vanhecke D., Hocevar S., Petri-Fink A., and Rothen-Rutishauser B. 2017. A novel technique to determine the cell type specific response within an in vitro co-culture model via multicolour flow cytometry. Scientific Reports Vol. 7 No. 434, doi:10.1038/s41598-017-00369-4.
Cossarizza A., et al. 2017. Guidelines for the use of flow cytometry and cell sorting in immunological studies. Eur. J. Immunol. Vol. 47, p. 1584-1797.
El-Kadiry AE-H., Rafei M., and Shammaa R. 2021. Cell Therapy: Types, regulation, and clinical benefit. Front. Med. Vol. 8: 756029.
Fajrial A.K., He Q.Q., Wirusanti N.I., Slansky J.E., and Ding X. 2020. A review of emerging physical transfection methods for CRISPR/Cas9-mediated gene editing. Theranostics Vol. 10 No. 12, p. 5532-5549.
Fus-Kujawa A., Prus P., Bajdak-Rusinek K., Teper P., Gawron K., Kowalczuk A., and Sieron A.L. 2021. An overview of methods and tools for transfection of eukaryotic cells in vitro. Front. Bioeng. Biotechnol. Vol. 9:701031.
Kim Y.-G. et al. 2012. New cell line development for antibody-producing Chinese hamster ovary cells using split green fluorescent protein. BMC Biotechnology Vol. 12:24.
Lai T., Yang Y., and Ng S.K. 2013. Advances in mammalian cell line development technologies for recombinant protein production. Pharmaceuticals Vol. 6, p. 579-603, doi:10.3390/ph6050579.
Lalonde M.-E., and Durocher Y. 2017. Therapeutic glycoprotein production in mammalian cells. J. Biotechnology Vol. 251, p. 128-140.
Leary J.F. 2005. Ultra high-speed sorting. Cytometry Part A Vol. 67A, p. 76-85, doi: 10.1002/cyto.a.20160.
Lennartz K., Lu M., Flasshove M., Moritz T., and Kirstein U. 2005. Improving the biosafety of cell sorting by adaptation of a cell sorting system to a biosafety cabinet. Cytometry Part A Vol. 66A, p. 119-127, doi:10.1002/cyto.a.20157.
Li F., Vijayasankaran N., Shen A., Kiss R., and Amanullah A. 2010. Cell culture processes for monoclonal antibody production. mAbs Vol. 2 No. 5, p. 466-477.
Marder P. et al. 1990. Selective cloning of hybridoma cells for enhanced immunoglobulin production using flow cytometric cell sorting and automated laser nephelometry. Cytometry Vol. 11, p. 498-505.
Noh S.M., Shin S., and Lee G.M. 2018. Comprehensive characterization of glutamine synthetase-mediated selection for the establishment of recombinant CHO cells producing monoclonal antibodies. Scientific Reports Vol. 8:5361, doi:10.1038/s41598-018-23720-9.
O'Brien A.A., Lee K., Fu, H.-Y., Lee Z., Le T.S., Stach C.S., McCann M.G., Zhang, A.Q., Smanski M.J., Somia, N.V., and Hu W.-S. 2018. Single copy transgene integration in a transcriptionally active site for recombinant protein synthesis. Biotechnol J. Vol. 13 No. 10: e1800226. doi:10.1002/biot.201800226.
Pereira H., Schulze P.S.C., Schüler L.M., Santos T., Barreira L., and Varela J. 2018. Fluorescence activated cell-sorting principles and applications in microalgal biotechnology. Algal Research Vol. 30, p. 113-120.
Scherdin U., Rhodes K., and Breindl M. 1990. Transcriptionally active genome regions are preferred targets for retrovirus integration. J. Virol. Vol. 64 No. 2, p. 907-912.
Umeyama R., Yamawaki T., Liu D., Kanazawa S., Takato T., Hoshi K., and Hikita A. 2020. Optimization of culture duration of bone marrow cells before transplantation with a β-tricalcium phosphate/recombinant collagen peptide hybrid scaffold. Regenerative Therapy Vol. 14, p. 284-295.
Wlaschin K.F. and Hu W.-S. 2006. Fedbatch culture and dynamic nutrient feeding. Adv Biochem Engin/Biotechnol Vol. 101, p. 43-74, doi:10.1007/10015.
Wurm F.M. 2004. Production of recombinant protein therapeutics in cultivated mammalian cells. Nat Biotech Vol. 22 No. 11, p. 1393-1398.

## Claims

1. A system comprising at least
(i) a cell culturing device,
(ii) a cell sorting device.

2. The system according to claim 1, further comprising
(iii) a direct connection from said cell culturing device to said cell sorting device, and/or
(iv) a direct connection from said cell sorting device to said cell culturing device; or further comprising
(iii) an indirect connection from said cell culturing device to said cell sorting device, and/or
(iv) an indirect connection from said cell sorting device to said cell culturing device.

3. The system according to any one of claims 1 or 2, wherein the cell culturing device and the cell sorting device are fitted in the same housing or apparatus.

4. The system according to any one of claims 1 - 3, wherein the system further comprises a device for genetic modification of cells by physical transfection, preferably by at least one of the methods selected from the group consisting of electroporation, sonoporation, magnetofection, microinjection, gene injection, laserfection, optical transfection and biolistic transfection, or by chemical transfection, preferably by at least one of the methods selected from the group consisting of calcium phosphate-based, cationic lipid-based, DEAE-dextran-based and nano-particle-based transfection, and/or by viral transfection (transduction), preferably by at least one of the methods selected from the group consisting of retroviral, lentiviral, herpes-viral, adenoviral, and adeno-associated viral based transfection;
preferably wherein the system further comprises a direct connection between said device for genetic modification of cells and the cell culturing device.

5. The system according to any one of claims 1 - 4, wherein the system further comprises a device to which the cell sorting device can separate out one or more single cell-sorted cells;
preferably wherein the system further comprises a direct or indirect connection between the cell sorting device and said device to which the cell sorting device can separate out one or more single cell-sorted cells.

6. The system according to any one of claims 1 - 5, wherein the system is a fully closed system and/or a system which is kept sterile inside,
and/or wherein said system is a partially or fully automated system.

7. A method for developing and/or optimizing a cell culture process, comprising at least the steps of
(a) growing a cell culture of a polyclonal population of cells in a cell culturing device,
(b) selecting cells in a cell sorting device,
(c) optionally repeating said steps (a) to (b) at least once;
preferably comprising at least the steps of
(a) growing a cell culture of a polyclonal population of cells in a cell culturing device,
(b) transferring cells from the cell culturing device into a cell sorting device by direct connection(s) from the cell culturing device to the cell sorting device,
(c) selecting cells in a cell sorting device,
(d) recirculating selected cells from the cell sorting device back to the cell culturing device by direct connection(s) from the cell sorting device to the cell culturing device,
(e) optionally repeating said steps (a) to (d) at least once.

8. The method for developing and/or optimizing a cell culture process according to claim 7, comprising prior to step (a) a further step of genetically modifying cells by physical transfection, preferably by at least one of the methods selected from the group consisting of electroporation, sonoporation, magnetofection, microinjection, gene injection, laserfection, optical transfection and biolistic transfection, and/or by chemical transfection, preferably by at least one of the methods selected from the group consisting of calcium phosphate-based, cationic lipid-based, DEAE-dextran-based and nano-particle-based transfection, and/or by
viral transfection (transduction), preferably by at least one of the methods selected from the group consisting of retroviral, lentiviral, herpes-viral, adenoviral, and adeno-associated viral based transfection;
preferably wherein said step of genetically modifying cells by physical transfection, chemical transfection and/or viral transfection (transduction) is performed in the device for genetic modification of cells according to claim 4, or in the cell culturing device of the system according to any one of claims 1 - 3.

9. The method according to any one of claims 7 and 8, wherein said cell culture process is suited for upscaling to large-scale manufacturing, and/or said cell culturing device is a small-scale cell culturing device;
preferably wherein said cell culture process is a batch culture, fed-batch culture, a continuous culture, and/or a perfusion culture process.

10. The method according to any one of claims 7 - 9, wherein the cells in said cell culturing device genetically modified by transfection, transduction and/or gene editing tools comprise at least one modified homologous and/or heterologous polynucleotide, preferably wherein said polynucleotide has been stably integrated in the genome of the cells, and/or preferably wherein said genetic modification is performed within said cell culturing device.

11. The method according to any one of claims 7 - 10, wherein, after performing all respective obligatory method steps at least once, the cells selected in the cell sorting device are subjected to single-cell cloning.

12. The system according to any one of claims 1 - 6, or the method according to any one of claims 7 - 11, wherein said cell culturing device is selected from the group consisting of a bioreactor, a stir-tank bioreactor, a spinner flask, a shaker flask, a shaker tube, a wave-mixing bioreactor, a petri dish, a multi-well cell culture plate and a microtiter plate;
preferably wherein said bioreactor is selected from the group consisting of an Ambr 15 cell culture bioreactor, an Ambr 250 cell culture bioreactor, an univessel bioreactor, a rocked motion bioreactor, a mobius bioreactor (Merck Millipore), a BioFlo bioreactor (Eppendorf), a Xcellerex bioreactor (Cytiva), a Biolector bioreactor (m2p-labs), a Multifors bioreactor (Infors), a DASbox bioreactor (Eppendorf), and a Micro-matrix bioreactor (Applikon);
and/or wherein said cell culturing device is a stainless steel cell culturing device, a synthetic or plastics cell culturing device, and/or a disposable cell culturing device.

13. The system according to any one of claims 1 - 6 and 12, or the method according to any one of claims 7 - 12, wherein said cell sorting device is a fluorescence-activated cell sorter (FACS), a magnetic activated cell sorter (MACS), a microchip-based cell sorting device, or a benchtop flow cytometry cell sorter.

14. The system according to any one of claims 2 - 6, 12 - 13, or the method according to any one of claims 7 - 13, wherein said direct connection(s) from said cell culturing device to said cell sorting device is suitable for transferring cells from said cell culturing device to said cell sorting device, and wherein said direct connection(s) from said cell sorting device to said cell culturing device is suitable for transferring cells from said cell sorting device to said cell culturing device;
and/or wherein said direct connection(s) from said cell culturing device to said cell sorting device, and said direct connection(s) from said cell sorting device to said cell culturing device, are selected from the group consisting of tubes, lines, flexible hoses, pipes or cables;
and/or wherein said direct connection(s) are comprising pumps for transferring the cells, preferably wherein the pumps are automatically controlled.

15. The system according to any one of claims 3 - 6, 12 - 14, or the method according to any one of claims 7 - 13, wherein said indirect connection(s) from said cell culturing device to said cell sorting device is an automatic pipetting robot, and/or wherein said indirect connection(s) from said cell sorting device to said cell culturing device is an automatic pipetting robot.

16. The system according to any one of claims 1 - 6, 12 - 15, or the method according to any one of claims 7 - 15, wherein said cell is selected from an animal cell, a mammalian cell, an insect cell, a plant cell, an algae cell and a fungus cell, preferably wherein said cell is a mammalian cell, further preferably wherein said cell is an immortalized cell line or a stem cell, more preferably wherein said cell is a human, simian or rodent cell line, most preferably wherein said cell line is selected from the group consisting of Chinese Hamster Ovary (CHO) cells, baby hamster kidney (BHK) cells, COS cells, mouse myeloma cells (NS0, SP2/0), human embryonic kidney (HEK) cells, human retina-derived cells (PER-C6), and human amniocyte cells (CAP cells).

17. The method according to any one of claims 7 - 16, wherein the culturing conditions for which the cell culture process is to be optimized are selected from the group consisting of culture medium, culture temperature, pH value, oxygen concentration, cell density, addition of cell nutrients including the time of addition and the target concentration of the nutrients in the culture medium, culturing time, specific power input, rate of gas supply, rate of perfusion, and osmolarity of the culture medium; and/or
wherein said selection of cells in the cell sorting device is performed according to target parameters selected from the group consisting of high cell viability, cell size, high and/or stable cell density, high and/or stable yield of a biomolecule of interest produced by the cells in the cell culture, and a desired property of a biomolecule of interest produced by the cells in the cell culture.

18. Use of the system according to any one of claims 1 - 6, 12 - 16 in a method for developing and/or optimizing a cell culture process according to any one of claims 7 - 17.

19. The method according to any one of claims 7 - 17, or the use of the system according to claim 18, wherein said cell culture process is suited for producing recombinant polypeptides, proteins, vaccines, and/or cellular biopharmaceuticals;
preferably wherein said proteins are selected from the group consisting of glycoproteins, antibodies, cytokines, receptors, fusion proteins, and viral proteins, or fragments thereof;
and/or
wherein said cellular biopharmaceuticals are selected from the group consisting of pluripotent stem cells (PSCs), including embryonic stem cells (ESCs), epiblast stem cells (EpiSCs), embryonic germ cells (EGCs), and induced pluripotent stem cells (iPSCs); adult stem cells (ASCs), including hematopoietic stem cells (HSCs), skin stem cells (SSCs), neural stem cells (NSCs), and mesenchymal stem cells (MSCs); and cancer stem cells (CSCs); non-stem cell-based cells and modified immune cells including tumor-infiltrating lymphocytes (TILs), tumor-specific T-cell receptor (TCR)-modified T cells, chimeric antigen receptor (CAR)-T cells, CAR-NK cells, lymphokine activated killer (LAK) cells, cytokine-induced killer (CIK) cells, γδ-T cells, and NK cells.

20. A system comprising more than one of the systems according to any one of claims 1 - 6, 12 - 16, wherein the system is suitable for developing and/or optimizing a particular polyclonal cell culture with regard to multiple target cell culture parameters in parallel, and/or for developing and/or optimizing different polyclonal cell cultures with regard to a particular target cell culture parameter, preferably wherein the system comprises a multitude of the systems according to any one of claims 1 - 6, 12 - 16.
